# EUROPÄISCHE PATENTSCHRIFT

(11) **EP 4 394 045 B1**
(45) Veröffentlichungstag und Bekanntmachung des Hinweises auf die Patenterteilung: **09.07.2025**
(21) Anmeldenummer: 22216869.2
(22) Anmeldetag: 28.12.2022
(51) Int. Cl.: C12Q 1/56, G01N 33/86

(54) **VERFAHREN ZUR BESTIMMUNG VON FIBRINOGEN**
METHOD FOR DETERMINING FIBRINOGEN
PROCÉDÉ DE DÉTERMINATION DU FIBRINOGÈNE

(43) Veröffentlichungstag der Anmeldung: 03.07.2024
(73) Patentinhaber: Siemens Healthcare Diagnostics Products GmbH, 35041 Marburg (DE)
(72) Erfinder: Patzke, Juergen, 35043 Marburg (DE)
(74) Vertreter: Siemens Healthineers Patent Attorneys

(56) Entgegenhaltungen:
- A CLAUSS: "Gerinnungsphysiologische Schnellmethode zur Bestimmung des Fibrinogens", vol. 17, no. 4, 1 January 1957 (1957-01-01), CH, pages 237 - 246, XP055385211, ISSN: 0001-5792, Retrieved from the Internet <URL:http://www.ncbi.nlm.nih.gov/pubmed/13434757> DOI: 10.1159/000205234
- THEUSINGER OLIVER ET AL: "In vitro factor XIII supplementation increases clot firmness in Rotation Thromboelastometry (ROTEM )", vol. 104, no. 08, 1 January 2010 (2010-01-01), DE, pages 385 - 391, XP093048716, ISSN: 0340-6245, Retrieved from the Internet <URL:https://www.thieme-connect.com/products/ejournals/pdf/10.1160/TH09-12-0858.pdf> DOI: 10.1160/TH09-12-0858

## Beschreibung

Die vorliegende Erfindung liegt auf dem Gebiet der Blutgerinnungsdiagnostik und betrifft ein verbessertes Verfahren zur quantitativen Bestimmung von Fibrinogen in einer Probe.

Fibrinogen ist die wasserlösliche Vorstufe von Fibrin, welches die Matrix für den Wundverschluss bildet. Die Gerinnungsprotease Thrombin (Faktor IIa) spaltet Fibrinogen und aktiviert auf diese Weise die Fibrinbildung, also die Gerinnselbildung. Erniedrigte Fibrinogenspiegel gehen mit einer Blutungsneigung einher. Akut erhöhte Fibrinogenspiegel findet man häufig bei Entzündungen, postoperativ und in anderen Situationen. Langfristig erhöhte Fibrinogenspiegel gelten als Risikoindikator für thrombotische Erkrankungen.

Im Stand der Technik sind eine Reihe verschiedener Methoden zur Bestimmung der Fibrinogenkonzentration bekannt.

In CA 1062501 ist ein Fibrinogenbestimmungsverfahren basierend auf der Messung der Thrombinzeit beschrieben, wobei die Thrombinzeit bekanntermaßen keine präzise Bestimmung der Fibrinogenkonzentration zulässt, weil neben Fibrinogen weitere Faktoren, wie z.B. Antikoagulanzien wie Heparin oder direkte Thrombin-Inhibitoren oder auch die Gegenwart von Fibrin- oder Fibrinogen-Spaltprodukten, die Thrombinzeit beeinflussen können, und die Thrombinzeit in der Regel nur bei schweren Fibrinogen-Mangelzuständen anspricht. Bei einem Thrombinzeitverfahren wird üblicherweise eine unverdünnte Plasmaprobe mit einer verhältnismäßig geringen Thrombinmenge zur Aktivierung der Gerinnung vermischt und die Fibrinbildung, also die Extinktionsänderung des Reaktionsansatzes photometrisch gemessen und anschließend die Gerinnungszeit bestimmt. Gemäß CA 1062501 wird jedoch keine Gerinnungszeit bestimmt, sondern es wird das Maximum der ersten Ableitung der Reaktionskurve oder mit anderen Worten die maximale Extinktionsänderung der Reaktionskurve bestimmt. Es wurde gefunden, dass die maximale Extinktionsänderung linear mit der Fibrinogenkonzentration korreliert, so dass Letztere mit Hilfe einer Kalibrationskurve, die aus einer Zuordnung von bekannten Fibrinogenkonzentrationen und maximalen Extinktionsänderungen erstellt wurde, bestimmt werden kann.

Ein wesentlich präziseres und häufig angewandtes Verfahren zur Bestimmung der Fibrinogenkonzentration ist die sogenannte Clauss-Methode (Clauss, A., Gerinnungsphysiologische Schnellmethode zur Bestimmung des Fibrinogens. 1957, Acta haemat. 17: 237-246). Der Test ist eine Variation der Thrombinzeit, bei der eine Plasmaprobe mit Thrombin als Gerinnungsaktivator vermischt wird und die Gerinnungszeit bestimmt wird. Bei der Clauss-Methode wird im Reaktionsansatz eine verhältnismäßig geringe Fibrinogenkonzentration mit einer verhältnismäßig hohen, standardisierten Thrombinkonzentration kombiniert, wodurch die Fibrinbildungsgeschwindigkeit praktisch ausschließlich mit der Fibrinogenkonzentration korreliert. Die verhältnismäßig geringe Fibrinogenkonzentration im Reaktionsansatz wird üblicherweise durch die Verwendung von vorverdünnten Plasmaproben hergestellt.

Im Reaktionsansatz wird dann die Fibrinbildung, also die Gerinnselbildung photometrisch bestimmt. Durch die Fibrinbildung trübt sich der Reaktionsansatz zunehmend, so dass durch eine Absorptionsmessung die Fibrinbildung quantitativ gemessen werden kann.

Üblicherweise wird dann die Gerinnungszeit der Probe bestimmt. Die Gerinnungszeit der Probe verhält sich proportional zur Fibrinogenmenge. Die Gerinnungszeit einer Probe ist die Zeit vom Zeitpunkt der Zugabe von Thrombin zur Probe bis zum Zeitpunkt der Messung einer fassbaren Fibrinbildung, also Trübung des Reaktionsansatzes. Dabei kann die "fassbare Fibrinbildung" als ein test- und gerätespezifischer Schwellenwert definiert sein, der -wenn er überschritten wird- die Gerinnungszeit angibt. Alternativ kann die "fassbare Fibrinbildung" ausgehend von der Signaldifferenz vor dem Start und nach Abschluss der Gerinnungsreaktion als ein test- und gerätespezifischer prozentualer Signaldifferenzwert definiert sein, der -wenn er erreicht wurde- die Gerinnungszeit angibt. Die Reaktionskinetik der Fibrinbildung in einem Clauss-Test unterscheidet sich erheblich von der Reaktionskinetik der Fibrinbildung in einem Thrombinzeit-Test. Beim Clauss-Test beginnt die Fibrinbildung je nach Fibrinogen-Konzentration bereits 3 Sekunden nach Zugabe des Thrombinreagenzes, also wesentlich früher als beim Thrombinzeit-Test, bei dem die Fibrinbildung frühestens nach 10 Sekunden einsetzt. Außerdem ist beim Clauss-Test die Fibrinbildungsgeschwindigkeit zumindest in verhältnismäßig hochkonzentrierten Proben höher als beim Thrombinzeit-Test. Die Reaktionskurven für Proben mit hoher Fibrinogenkonzentration beim Clauss-Test zeichnen sich im Vergleich zum Thrombinzeit-Test daher durch eine kurze Lag-Phase, einen steilen Anstieg und eine relativ schnell erreichte Plateauphase aus. Die Reaktionskurven für Proben mit niedriger Fibrinogenkonzentration beim Clauss-Test zeigen im Vergleich zum Thrombinzeit-Test ebenfalls eine kurze Lag-Phase, einen flachen Anstieg und eine so späte Plateauphase, dass diese meist erst nach Abschluss der Messung eintritt. Die Plateauphase ist bei der Thrombinzeit dagegen deutlich schwächer ausgeprägt oder sie entfällt sogar komplett.

Ein anderes Verfahren zur Bestimmung von Fibrinogen ist die Bestimmmung des sog. "abgeleiteten Fibrinogens", also die aus der Bestimmung der Thromboplastinzeit abgeleitete Bestimmung des Fibrinogens. Dazu wird eine Plasmaprobe mit einem Thromboplastin (z.B. lipidierter Gewebefaktor) als Gerinnungsaktivator vermischt und aus der nephelometrisch oder turbidimetrisch gemessenen Fibrinbildungskurve wird die Fibrinogenkonzentration (abgeleitetes Fibrinogen) kalkuliert.

Die beschriebenen bekannten Verfahren, insbesondere die Clauss-Methode, haben jedoch den Nachteil, dass der Messbereich stark limitiert ist. Da bei der Clauss-Methode die Probe mit einer verhältnismäßig hohen Thrombinkonzentration in Kontakt gebracht werden muss, damit die Fibrinbildungsgeschwindigkeit praktisch ausschließlich mit der Fibrinogenkonzentration korreliert, hat dies den Nachteil, dass bereits Proben mit leicht erhöhtem Fibrinogengehalt eine extrem schnell einsetzende Gerinnung aufweisen. So können Proben mit einem Fibrinogengehalt von etwa 5 g/L (Normalbereich: 1,8-3,5 g/L) eine Gerinnungszeit von nur 3,8 Sekunden aufweisen, eine Zeitspanne, die selbst für automatisierte Analysesysteme nur schwer zu erfassen ist. Proben mit noch höheren Fibrinogenkonzentrationen können nur zuverlässig analysiert werden, wenn sie vor der Messung verdünnt werden (z.B. 1:10 oder 1:100 in Puffer). Würde jedoch jede Probe generell vor der Messung so verdünnt werden, dass der Messbereich im oberen Fibrinogenkonzentrationsbereich erweitert wird, würde dies jedoch automatisch zu einer Begrenzung des Messbereichs im unteren Fibrinogenkonzentrationsbereichs führen, da bei Proben mit vermindertem Fibrinogengehalt nur noch so wenig Fibrinogen im Reaktionsansatz vorhanden ist, dass nur eine unzureichende oder gar keine nachweisbare Gerinnselbildung mehr stattfinden kann. Typischerweise müssen daher viele Proben mehrfach gemessen werden, weil nach der ersten Messung weitere Messungen bei anderen Probenverdünnungen durchgeführt werden müssen. Dieses Vorgehen ist zeit- und kostenaufwändig.

Der vorliegenden Erfindung liegt daher die Aufgabe zugrunde ein Verfahren zur Bestimmung der Fibrinogenkonzentration bereitzustellen, das -gegenüber den bekannten Verfahren zur Bestimmung von Fibrinogen, bei denen ein Reaktionsgemisch durch Vermischen einer typischerweise verdünnten Probe mit mindestens einem Gerinnungsaktivator bereitgestellt wird und die Fibrinbildung in dem Reaktionsgemisch gemessen wird- in einer einzigen Messung einen erweiterten Messbereich abdeckt und damit die Anzahl von notwendigen Mehrfachmessungen deutlich vermindert.

Es wurde gefunden, dass die Zugabe von Faktor XIII zu der Probe bzw. zu dem Reaktionsgemisch aus Probe und Gerinnungsaktivator, den Messbereich im unteren Fibrinogenkonzentrationsbereich erweitert. Dies bewirkt wiederum, dass in vielen Fällen auf eine Mehrfachmessung einer Probe mit vermindertem Fibrinogengehalt verzichtet werden kann.

Faktor XIII ist bekannt als das Enzym, welches bei der Gerinnselbildung die wichtige Rolle der Quervernetzung der Fibrinfäden übernimmt. Durch diese Quervernetzung wird das Gerinnsel stabilisiert. Im Plasma gesunder Individuen findet sich typischerweise ein Faktor XIII-Gehalt von 70 bis 140 %.

Gegenstand der vorliegenden Erfindung ist also ein Verfahren zur Bestimmung von Fibrinogen in einer Probe, das folgende Schritte umfasst:
a) Bereitstellen eines Reaktionsgemischs durch Vermischen der Probe mit mindestens einem Gerinnungsaktivator und
b) Messen der Fibrinbildung in dem Reaktionsgemisch,
wobei dem Reaktionsgemisch zusätzlich Faktor XIII zugesetzt wird.

Bevorzugterweise wird dem Reaktionsgemisch eine Menge von Faktor XIII zugesetzt, so dass die Endkonzentration von zugesetztem Faktor XIII in dem Reaktionsgemisch 5 bis 200 % der Norm entspricht. 100 % der Norm entspricht der Faktor XIII-Aktivität, die in einem Plasmapool von mehreren gesunden Spendern gemessen wird.

Dem Reaktionsgemisch kann beispielsweise isolierter humaner Faktor XIII (gereinigt aus humanem Plasma oder synthetisch erzeugt) oder rekombinanter Faktor XIII (gentechnologisch gewonnen) zugesetzt werden.

Der zugesetzte Faktor XIII kann in Form des (durch Thrombin) aktivierbaren Proenzyms (Faktor XIII) oder als bereits aktiviertes Enzym (Faktor XIIIa) zugesetzt werden.

Vorzugsweise wird Faktor XIII mit der Probe vermischt, bevor anschließend der Gerinnungsaktivator dem Reaktionsgemisch zugegeben wird. Alternativ können der Faktor XIII und der Gerinnungsaktivator gleichzeitig mit der Probe vermischt werden, z.B. indem ein Reagenz enthaltend Faktor XIII und den Gerinnungsaktivator der Probe zugegeben wird.

Als Gerinnungsaktivator eignet sich jede Substanz oder jedes Substanzgemisch, die/das bei Zugabe zu einer humanen Plasmaprobe den extrinsischen und/oder intrinsischen Weg des Blutgerinnungssystems aktiviert. Bevorzugte Gerinnungsaktivatoren sind Gemische aus Gewebefaktor (tissue factor) und Phospholipiden (Thromboplastine) und Calciumchlorid. Der Gewebefaktor kann aus Hirn, Lunge oder Plazentagewebe eines Säugetiers stammen (z.B. Mensch oder Kaninchen), oder er kann alternativ rekombinant oder synthetisch hergestellt sein. Ein anderer bevorzugter Gerinnungsaktivator ist Thrombin, beispielsweise humanes oder bovines Thrombin. Alternativ können auch gerinnungsaktivierende Schlangengifte oder eine aus Schlangengift isolierte gerinnungsaktivierende Protease als Aktivator eingesetzt werden. Weitere Gerinnungsaktivatoren sind Phospholipide und negativ geladene Oberflächen wie Glas, Silica, Kaolin, Ellagsäure, Celit und Plättchenfaktor 3.

Unter dem Begriff "Probe" ist eine Plasma- oder Vollblut-Probe zu verstehen, vorzugsweise eine humane oder tierische Plasma- oder Vollblut-Probe. Plasma- oder Vollblut-Proben können ein Antikoagulanz wie Citrat oder EDTA enthalten, welches bei der Probenentnahme zur Vermeidung einer spontanen, unkontrollierten Gerinnung der Probe zugegeben wird.

Das Messen der Fibrinbildung in dem Reaktionsgemisch erfolgt vorzugsweise durch die Messung der Extinktionswerte des Reaktionsgemischs über die Zeit. Die Messung der Extinktionswerte kann photometrisch, d.h. durch die Messung der Lichtabschwächung eines durch das Reaktionsgemisch gesendeten Lichtstrahls, oder nephelometrisch, d.h. durch die Messung von Streulichtanteilen eines durch das Reaktionsgemisch gesendeten Lichtstrahls, erfolgen. Idealerweise wird mit der Messung unmittelbar nach der Zugabe des Gerinnungsaktivators zum Reaktionsgemisch begonnen, und die Messung der Extinktionswerte wird kontinuierlich bis zum Abschluss der Fibrinbildung durchgeführt.

Die quantitative Fibrinbestimmung erfolgt anschließend durch Auswertung der ermittelten Extinktionskurve mit einem dem Fachmann geläufigen Auswerteverfahren, wie z.B. der Bestimmung der maximalen Extinktionsänderung und dem Vergleich mit einer Kalibrationskurve, die aus einer Zuordnung von bekannten Fibrinogenkonzentrationen und maximalen Extinktionsänderungen erstellt wurde.

Ein weiterer Gegenstand der vorliegenden Erfindung ist ein Reagenz zur Verwendung in einem Verfahren zur Bestimmung von Fibrinogen in einer Probe, welches mindestens einen Gerinnungsaktivator und Faktor XIII enthält. Das Reagenz ermöglicht eine besonders einfache Bereitstellung eines Reaktionsgemischs für die erfindungsgemäße Bestimmung von Fibrinogen.

Der in dem Reagenz enthaltende Gerinnungsaktivator kann -wie bereits oben beschrieben- jede Substanz oder jedes Substanzgemisch sein, die/das bei Zugabe zu einer humanen Plasmaprobe den extrinsischen und/oder intrinsischen Weg des Blutgerinnungssystems aktiviert. Bevorzugterweise enthält das Reagenz einen Gerinnungsaktivator aus der Gruppe Thrombin und Thromboplastin.

Der in dem Reagenz ferner enthaltene Faktor XIII kann -wie bereits oben beschrieben- isolierter humaner Faktor XIII oder rekombinanter Faktor XIII sein und entweder in Form des aktivierbaren Proenzyms (Faktor XIII) oder als bereits aktiviertes Enzym (Faktor XIIIa) eingesetzt werden.

Noch ein weiterer Gegenstand der vorliegenden Erfindung ist ein Testkit zur Verwendung in einem Verfahren zur Bestimmung von Fibrinogen in einer Probe. Das erfindungsgemäße Testkit umfasst dazu i) ein erstes Reagenz enthaltend mindestens einen Gerinnungsaktivator und ii) ein zweites Reagenz enthaltend Faktor XIII. Der in dem ersten Reagenz enthaltene Gerinnungsaktivator und der in dem zweiten Reagenz enthaltene Faktor XIII können in ihren jeweiligen Ausführungsformen wie weiter oben beschrieben ausgestaltet sein.

### FIGURENBESCHREIBUNG

- FIG. 1A: zeigt eine Kalibrationskurve zur Ermittlung der Fibrinogen-Konzentration, die gemäß dem Stand der Technik ohne Zugabe von Faktor XIII zum Reaktionsansatz ermittelt wurde.
- FIG. 1B: zeigt eine Kalibrationskurve zur Ermittlung der Fibrinogen-Konzentration, die erfindungsgemäß mit Zugabe von Faktor XIII zum Reaktionsansatz ermittelt wurde.
- FIG. 2A: zeigt die gemäß dem Stand der Technik ermittelten Fibrinogen-Konzentrationen (g/L) von Proben mit bekannter Fibrinogen-Konzentration, die ohne Zugabe von Faktor XIII zum Reaktionsansatz ermittelt wurden.
- FIG. 2B: zeigt die erfindungsgemäß ermittelten Fibrinogen-Konzentrationen (g/L) von Proben mit bekannter Fibrinogen-Konzentration, die mit Zugabe von Faktor XIII zum Reaktionsansatz ermittelt wurden.

### BEISPIELE

### BEISPIEL 1: Fibrinogentest nach Clauss in Gegenwart von exogenem Faktor XIII

Das Faktor XIII-Konzentrat Fibrogammin 250 (CSL Behring, Marburg, Deutschland) wurde in einer wässrigen Lösung mit 9 g/L Albumin, 8 g/L NaCL und 5 g/L Glukose aufgenommen. Die Faktor XIII-Konzentration in dieser Lösung betrug 7100 % der Norm.

Diese Faktor XIII-enthaltende Lösung wurde mit einem Reagenz vermischt, das Thrombin als Gerinnungsaktivator enthält (Dade Thrombin Reagenz, Siemens Healthineers, Marburg, Deutschland). Die obige wässrige Lösung wurde ebenfalls für die Herstellung einer Mischung ohne Faktor XIII verwendet. In Tabelle 1 sind die Mischungsverhältnisse der verschiedenen Komponenten gezeigt.

**Tabelle 1**

| | Wässrige Lösung ohne Faktor XIII [µL] | Wässrige Lösung mit Faktor XIII (7100 % der Norm) [µL] | Thrombin Reagenz [µL] | Faktor XIII-Konzentration in der Thrombin-Reagenzmischung [% der Norm] |
|---|---|---|---|---|
| Ohne Faktor XIII | 197,3 | 0 | 2802,7 | 0 |
| Mit Faktor XIII | 98, 65 | 98, 65 | 2802,7 | 233,5 |

Zur Bestimmung von Fibrinogen in humanen Plasmaproben (Standard Humanplasma) wurden die Reaktionsgemische wie folgt angesetzt:
24 µL Probe (1:3 vorverdünnt mit Owrens Veronal Puffer)
10 Sekunden Inkubation bei 37 °C
+ 76 µL Owrens Veronal Puffer
180 Sekunden Inkubation bei 37 °C
+ 50 µL Thrombin-Reagenzmischung mit/ohne Faktor XIII.

Die Faktor XIII-Konzentration in den so hergestellten Reaktionsgemischen betrug 77,8 % der Norm bzw. 0 % der Norm (ohne Zugabe von Faktor XIII).

In dem Reaktionsgemisch wurde kontinuierlich die Absorption bei 405 nm gemessen, und es wurde die Reaktionsgeschwindigkeit (mA/s) im Bereich zwischen 0 und 70 Sekunden ermittelt.

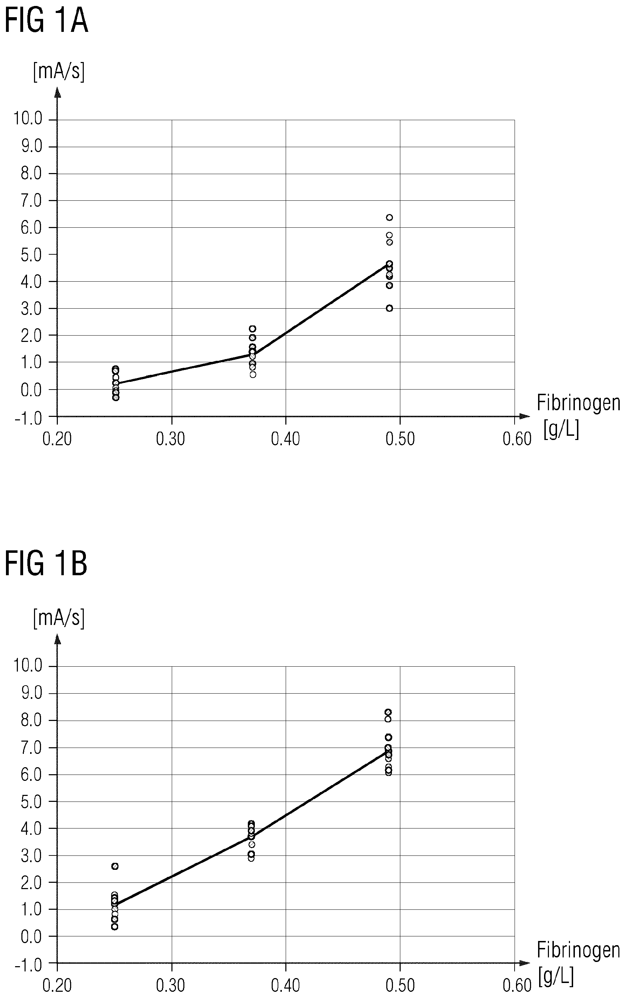

Zunächst wurden zwei Kalibrationskurven ermittelt. Dazu wurden mit Owrens Veronal Puffer verdünnte Standard Humanplasmaproben mit Fibrinogenkonzentrationen von 0,25, 0,37 und 0,49 g/L jeweils 10fach in einem Testansatz zur Fibrinogenbestimmung ohne Faktor XIII-Zugabe (Stand der Technik, FIG. 1A) und in einem Testansatz zur Fibrinogenbestimmung mit Faktor XIII-Zugabe (erfindungsgemäß, FIG. 1B) gemessen. Unter Berücksichtigung des deklarierten Fibrinogen-Wertes in Standard Humanplasma wurden die Konzentrationsstufen 0,25, 0,37 und 0,49 g/L Fibrinogen hergestellt. Die Mittelwerte aus den 10fach-Bestimmungen dienen als Stützpunkte der Kalibrationskurven (FIG. 1A und 1B).

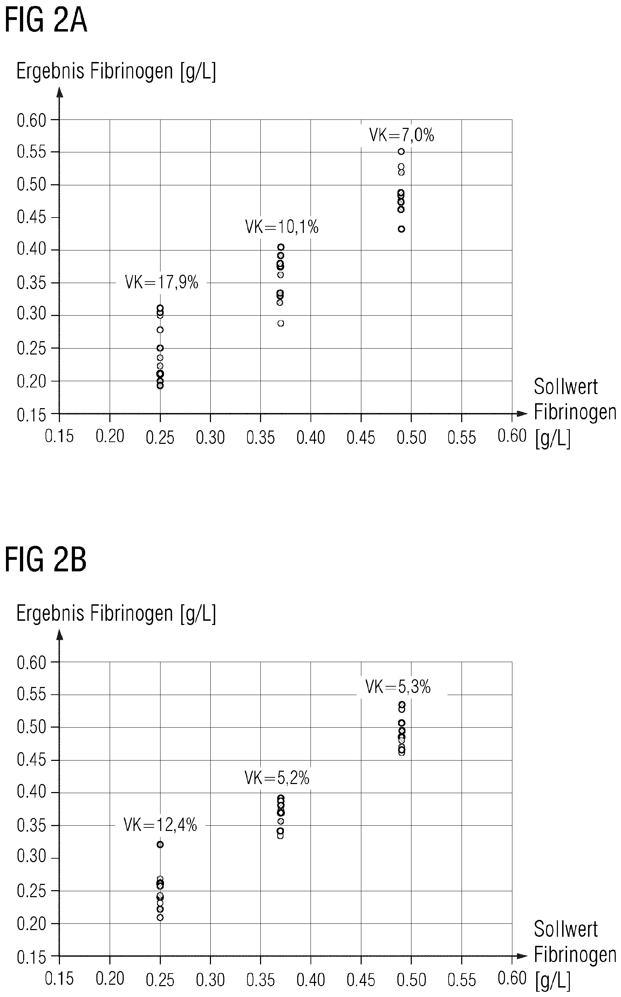

Anschließend wurden drei Plasmaproben mit bekannten Fibrinogenkonzentrationen (0,25, 0,37 und 0,49 g/L) jeweils 10fach in einem Testansatz zur Fibrinogenbestimmung ohne Faktor XIII-Zugabe (Stand der Technik, FIG. 2A) und in einem Testansatz zur Fibrinogenbestimmung mit Faktor XIII-Zugabe (erfindungsgemäß, FIG. 2B) gemessen. Die Rohwerte wurden unter Verwendung der Kalibrationskurven aus FIG. 1 ausgewertet, und es wurde der Variationskoeffizient für jede 10fach-Bestimmung ermittelt.

Der FIG. 1A ist entnehmbar, dass ohne Zugabe von Faktor XIII der untere Kalibrationspunkt von 0,25 g/L Fibrinogen sehr nahe an der Reaktionsgeschwindigkeit von 0 mA/s liegt. Einzelne Werte liegen im negativen Bereich (abfallende Exktinktion). Nur durch die Mittelwertbildung von 10 Bestimmungen ergibt sich ein positiver Wert von 0,2 mA/s. Es ist erkennbar, dass eine klare statistische Differenzierung des Kalibrationspunktes bei 0,25 g/L von dem bei 0,37 g/L Fibrinogen nicht möglich ist, da die Rohwerte einen Überlappungsbereich aufweisen. Wird hingegen Faktor XIII zugegeben, steigt das Niveau der Steigungswerte (mA/s) an, und der Kalibrationspunkt bei 0,37 g/L ist klar differenzierbar von dem Kalibrationspunkt bei 0,25 g/L, da es keinen Überlappungsbereich mehr gibt (FIG. 1B).

In FIG. 2 ist die Auswertung der gemessenen Rohwerte unter Verwendung der Kalibrationskurven aus FIG. 1 dargestellt. Die Probe mit dem Sollwert 0,25 g/L Fibrinogen weist ohne Zugabe von Faktor XIII einen weiten Streuungsbereich auf, der mit dem Streuungsbereich von der Probe mit dem Sollwert von 0,37 g/L Fibrinogen überlappt (FIG. 2A). Bei Zugabe von Faktor XIII ist die Streuung der Werte geringer, und die Probe mit 0,25 g/L Fibrinogen ist klar differenzierbar von der Probe mit 0,37 g/L Fibrinogen (FIG. 2B). Die Variationskoeffizienten sind bei Zugabe von Faktor XIII wesentlich geringer als ohne Zugabe von Faktor XIII.

In diesem Beispiel bewirkt die Zugabe von Faktor XIII ein unteres Messbereichsende von 0,25 g/L Fibrinogen, während ohne die Zugabe von Faktor XIII nur ein unterer Messbereich von 0,37 g/L Fibrinogen möglich wäre. Die Zugabe von Faktor XIII zum Reaktionsgemisch ermöglicht also eine Erweiterung des Messbereichs, so dass mit dem erfindungsgemäßen Verfahren die Fibrinogen-Konzentration in mehr Proben mit geringer Fibrinogen-Konzentration zuverlässig bestimmt werden kann.

## Patentansprüche

1. Verfahren zur Bestimmung von Fibrinogen in einer Probe, das Verfahren umfassend die Schritte
a) Bereitstellen eines Reaktionsgemischs durch Vermischen der Probe mit mindestens einem Gerinnungsaktivator und
b) Messen der Fibrinbildung in dem Reaktionsgemisch,
**dadurch gekennzeichnet, dass** dem Reaktionsgemisch zusätzlich Faktor XIII zugesetzt wird.

2. Verfahren gemäß Anspruch 1, wobei dem Reaktionsgemisch eine Menge von Faktor XIII zugesetzt wird, so dass die Endkonzentration von zugesetztem Faktor XIII in dem Reaktionsgemisch 5 bis 200 % der Norm entspricht.

3. Verfahren gemäß einem der vorhergehenden Ansprüche, wobei der Gerinnungsaktivator ausgewählt ist aus der Gruppe Thrombin und Thromboplastin.

4. Verfahren gemäß einem der vorhergehenden Ansprüche, wobei der zusätzlich zugesetzte Faktor XIII isolierter humaner Faktor XIII oder rekombinanter Faktor XIII ist.

5. Verfahren gemäß einem der vorhergehenden Ansprüche, wobei der zusätzlich zugesetzte Faktor XIII aktivierter Faktor XIIIa ist.

6. Reagenz zur Verwendung in einem Verfahren zur Bestimmung von Fibrinogen in einer Probe, das Reagenz enthaltend mindestens einen Gerinnungsaktivator und Faktor XIII.

7. Reagenz gemäß Anspruch 6, wobei der Gerinnungsaktivator ausgewählt ist aus der Gruppe Thrombin und Thromboplastin.

8. Reagenz gemäß einem der Ansprüche 6 und 7, wobei der Faktor XIII isolierter humaner Faktor XIII oder rekombinanter Faktor XIII ist.

9. Reagenz gemäß einem der Ansprüche 6 bis 8, wobei der Faktor XIII aktivierter Faktor XIIIa ist.

10. Testkit zur Verwendung in einem Verfahren zur Bestimmung von Fibrinogen in einer Probe, das Testkit umfassend
i) ein erstes Reagenz enthaltend mindestens einen Gerinnungsaktivator und
ii) ein zweites Reagenz enthaltend Faktor XIII.

11. Testkit gemäß Anspruch 10, wobei das erste Reagenz einen Gerinnungsaktivator aus der Gruppe Thrombin und Thromboplastin enthält.

12. Testkit gemäß einem der Ansprüche 10 und 11, wobei das zweite Reagenz isolierten humanen Faktor XIII oder rekombinanten Faktor XIII enthält.

13. Testkit gemäß einem der Ansprüche 10 bis 12, wobei der Faktor XIII im zweiten Reagenz aktivierter Faktor XIIIa ist.

## Claims

1. Method for determining fibrinogen in a sample, the method comprising the steps of
a) providing a reaction mixture by mixing the sample with at least one coagulation activator and
b) measuring fibrin formation in the reaction mixture,
**characterized in that** factor XIII is additionally added to the reaction mixture.

2. Method according to Claim 1, wherein there is added to the reaction mixture an amount of factor XIII such that the final concentration of added factor XIII in the reaction mixture corresponds to 5% to 200% of the norm.

3. Method according to either of the preceding claims, wherein the coagulation activator is selected from the group consisting of thrombin and thromboplastin.

4. Method according to any of the preceding claims, wherein the factor XIII additionally added is isolated human factor XIII or recombinant factor XIII.

5. Method according to any of the preceding claims, wherein the factor XIII additionally added is activated factor XIIIa.

6. Reagent for use in a method for determining fibrinogen in a sample, the reagent containing at least one coagulation activator and factor XIII.

7. Reagent according to Claim 6, wherein the coagulation activator is selected from the group consisting of thrombin and thromboplastin.

8. Reagent according to either of Claims 6 and 7, wherein the factor XIII is isolated human factor XIII or recombinant factor XIII.

9. Reagent according to any of Claims 6 to 8, wherein the factor XIII is activated factor XIIIa.

10. Test kit for use in a method for determining fibrinogen in a sample, the test kit comprising
i) a first reagent containing at least one coagulation activator and
ii) a second reagent containing factor XIII.

11. Test kit according to Claim 10, wherein the first reagent contains a coagulation activator from the group consisting of thrombin and thromboplastin.

12. Test kit according to either of Claims 10 and 11, wherein the second reagent contains isolated human factor XIII or recombinant factor XIII.

13. Test kit according to any of Claims 10 to 12, wherein the factor XIII in the second reagent is activated factor XIIIa.

## Revendications

1. Procédé de détermination du fibrinogène dans un échantillon, le procédé comprenant les stades
a) se procurer un mélange réactionnel par mélange de l'échantillon avec au moins un activateur de coagulation et
b) mesurer la formation de fibrine dans le mélange réactionnel,
**caractérisé en ce que** l'on ajoute supplémentairement du facteur XIII au mélange réactionnel.

2. Procédé suivant la revendication 1, dans lequel on ajoute au mélange réactionnel une quantité de facteur XIII telle, que la concentration finale du facteur XIII ajouté dans le mélange réactionnel représente de 5 à 200 % de la norme.

3. Procédé suivant l'une des revendications précédentes, dans lequel l'on choisit l'activateur de coagulation dans le groupe de la thrombine et de la thromboplastine.

4. Procédé suivant l'une des revendications précédentes, dans lequel le facteur XIII ajouté supplémentairement est du facteur XIII humain isolé ou du facteur XIII recombinant.

5. Procédé suivant l'une des revendications précédentes, dans lequel le facteur XIII ajouté supplémentairement est du facteur XIIIa activé.

6. Réactif à utiliser dans un procédé de détermination du fibrinogène dans un échantillon, le réactif contenant au moins un activateur de coagulation et du facteur XIII.

7. Réactif suivant la revendication 6, dans lequel l'activateur de coagulation est choisi dans le groupe de la thrombine et de la thromboplastine.

8. Réactif suivant l'une des revendications 6 et 7, dans lequel le facteur XIII ajouté supplémentairement est du facteur XIII humain isolé ou du facteur XIII recombinant.

9. Réactif suivant l'une des revendications 6 à 8, dans lequel le facteur XIII est du facteur XIIIa activé.

10. Trousse de test à utiliser dans un procédé de détermination du fibrinogène dans un échantillon, la trousse de test comprenant
i) un premier réactif contenant au moins un activateur de coagulation et
ii) un deuxième réactif contenant du facteur XIII.

11. Trousse de test suivant la revendication 10, dans laquelle le premier réactif contient un activateur de coagulation choisi dans le groupe de la thrombine et de la thromboplastine.

12. Trousse de test suivant l'une des revendications 10 et 11, dans laquelle le deuxième réactif contient du facteur XIII humain isolé ou du facteur XIII recombinant.

13. Trousse de test suivant l'une des revendications 10 à 12, dans laquelle le facteur XIII dans le deuxième réactif est du facteur XIIIa activé.
